(19) Europäisches Patentamt
European Patent Office
Office européen des brevets

(11) Veröffentlichungsnummer : **0 055 689**
**B1**

(12) # EUROPÄISCHE PATENTSCHRIFT

(45) Veröffentlichungstag der Patentschrift : **04.07.84**

(51) Int. Cl.³ : **C 07 C103/24**, C 07 C103/26, A 23 L 1/236

(21) Anmeldenummer : **81730123.7**

(22) Anmeldetag : **17.12.81**

(54) **In 3-Stellung substituierte 2,4,6-trihalogenierte Benzamide und deren Salze, deren Herstellung und Verwendung als Ersatzstoffe für natürliche Süssstoffe sowie Süssungsmittel auf Basis dieser Verbindungen.**

(30) Priorität : **22.12.80 DE 3048918**
**12.11.81 DE 3145395**

(43) Veröffentlichungstag der Anmeldung : **07.07.82 Patentblatt 82/27**

(45) Bekanntmachung des Hinweises auf die Patenterteilung : **04.07.84 Patentblatt 84/27**

(84) Benannte Vertragsstaaten :
**AT BE CH DE FR GB IT LI LU NL SE**

(56) Entgegenhaltungen :
**GB-A- 1 080 496**
**GB-A- 1 340 749**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Gries, Heinz, Dr.**
**Helmstedter Strasse 19**
**D-1000 Berlin 31 (DE)**
Erfinder : **Mützel, Wolfgang, Dr.**
**Weddigenweg 74**
**D-1000 Berlin 45 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 055 689 B1

Jouve, 18, rue St-Denis, 75001 Paris, France

**Beschreibung**

Die Erfindung betrifft in 3-Stellung substituierte 2,4,6-trihalogenierte Benzamide der allgemeinen Formel Ia

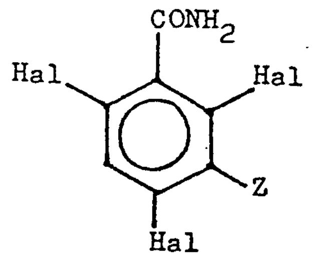

(Ia),

worin

Hal ein Chlor- oder Bromatom ist und Z eine Carboxylgruppe bedeutet, oder die Gruppe

$$-O_P-(CH_2)_K-O_L-(CH_2)_M-(\overset{X}{\overset{|}{C}}H)_N-\overset{Y}{\overset{|}{C}}H-COOH$$

darstellt, wobei

P und L die Zahlen 0 oder 1 sind, jedoch L die Zahl 0 bedeutet, wenn P oder K = 0 sind,
K die Zahlen 0 ; 2 bis 4,
M die Zahlen 0 ; 1 bis 3,
N die Zahlen 0 oder 1,
X ein Wasserstoffatom,
Y ein Wasserstoffatom oder eine niedere Alkylgruppe mit 1 bis 4 Kohlenstoffatomen, oder, wenn M oder N eine Zahl grösser 0 bedeutet, Y auch eine gegebenenfalls verätherte oder veresterte Hydroxylgruppe darstellt, oder wenn N die Zahl 1 bedeutet und M grösser als die Zahl 0 ist, X und Y zusammen eine zusätzliche Kohlenstoffbindung bilden, oder worin Hal ein Jodatom darstellt, Z eine Carboxylgruppe oder die Gruppe

$$-O_P-(CH_2)_K-O_L-(CH_2)_M-(\overset{X}{\overset{|}{C}}H)_N-\overset{Y}{\overset{|}{C}}H-COOH$$

bedeutet
und, wenn P und L die Zahl 0 bedeuten
K die Zahlen 0 ; 2 bis 4,
M die Zahlen 0 ; 1 bis 3,
N die Zahlen 0 oder 1,
X ein Wasserstoffatom,
Y ein Wasserstoffatom, eine niedere Alkylgruppe mit 1 bis 4 Kohlenstoffatomen oder eine gegebenenfalls verätherte oder veresterte Hydroxylgruppe darstellt oder, wenn N die Zahl 1 und M grösser als 0 ist,
X und Y zusammen eine zusätzliche Kohlenstoffbindung bilden,
und, wenn P und L die Zahl 1 bedeuten,
K die Zahlen 2 bis 4,
M die Zahlen 0 ; 1 bis 3
N die Zahl 0 oder 1
X ein Wasserstoffatom und
Y ein Wasserstoffatom oder eine niedere Alkylgruppe mit 1 bis 4 Kohlenstoffatomen darstellt,
und, wenn P die Zahl 1 und L die Zahl 0 bedeutet,
K, M und N die Zahl 0 darstellen und
Y ein Wasserstoffatom ist,
und, wenn P die Zahl 1 und L die Zahl 0 bedeutet,
K die Zahl 0,

M die Zahl 1 oder 2,
N die Zahl 0,
Y einen niederen Alkylrest mit 1 bis 4 Kohlenstoffatomen darstellt,
und, wenn P die Zahl 1 und L die Zahl 0 bedeutet,
K die Zahlen 2 bis 4,
M die Zahlen 0 ; 1 bis 3,
N die Zahl 1,
X ein Wasserstoffatom und
Y ein Wasserstoffatom oder einen niederen Alkylrest mit 1 bis 4 Kohlenstoffatomen bedeutet,
sowie deren Salze mit anorganischen Basen.

Als niedere Alkylgruppen Y sind der Methyl- und Äthylrest bevorzugt.

Bedeutet Y eine gegebenenfalls verätherte Hydroxylgruppe, so ist sie mit einem Alkylrest mit 1 bis 6 Kohlenstoffatomen veräthert, wobei der Methyl-, Äthyl- und n-Pentylrest bevorzugt sind.

Stellt Y eine veresterte Hydroxygruppe dar, so ist sie mit einer niederen Carbonsäure mit 1 bis 3 Kohlenstoffatomen im Alkylrest verestert, wobei die Essigsäure und Propionsäure bevorzugt sind.

Bedeutet Z—COOH oder die Gruppe

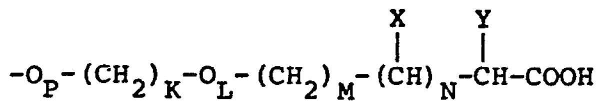

so sind folgende Reste bevorzugt :

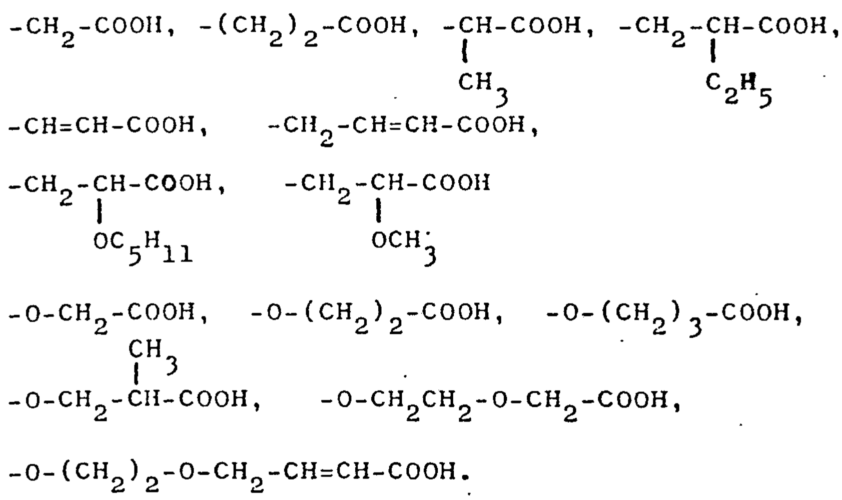

Verbindungen der allgemeinen Formel I, worin Hal ein Jodatom und Z die Gruppe

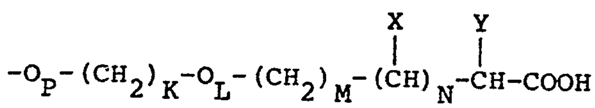

bedeutet und worin
P = 1 ; K, L und M = 0 ist
und
N = 0
Y = $—CH_3$, $—C_2H_5$, $—C_4H_9$, -Phenyl
oder
N = 1 oder 2,
X und Y je ein Wasserstoff
bedeutet sowie deren Salze mit Basen sind in der DAS 1 568 959 beschrieben und sind als Röntgenkontrastmittel in der Cholecystographie und als Choleretika geeignet.

Die neuen Verbindungen der Formel Ia sind thermisch stabile kristalline Verbindungen und besitzen sowohl als Saüre als auch in Form ihrer neutralen Salze mit anorganischen Basen eine ausserordentlich starke Süsskraft.

Die sogenannten synthetisch hergestellten « Süsstoffe » mit weit höherem Süssungsgrad als Rohrzucker (Saccharose) sind seit langem bekannt.

Die bekanntesten Vertreter dieser Substanzklasse sind Saccharin, Dulcin und Natriumcyclamat sowie Aspartame® und 2-Amino-4-nitro-1-phenyl-n-propyl-äther (Ultrasüss, P 4 000).

Die synthetischen Süssstoffe sollen neben einem hohen Süssungsgrad (geringe Substanzbelastung) keine Nebenwirkung und eine sehr gute Verträglichkeit besitzen. Ausserdem müssen sie die thermischen Belastungen beim Koch- oder Backvorgang unverändert überstehen können. Mangelnde Hitzebeständigkeit schränkt beispielsweise den Anwendungsbereich von Aspartame® stark ein ; und die gegen Dulcin bestehenden gesundheitlichen Bedenken bewirkten ein Verbot der Verwendung von Dulcin beispielsweise in der Bundesrepublik Deutschland und in den USA.

Der Bedarf an synthetischen Süssstoffen mit stärkerem Süssungsgrad und weiter verbesserter Allgemeinverträglichkeit ist aus medizinischen Gründen (Diabetiker) und diätetischen Gründen (Verringerung der Kalorienmenge) gross.

Die neuen erfindungsgemässen Verbindungen der Formel Ia besitzen die von synthetischen Süssstoffen geforderten Eigenschaften in überraschend hohem Masse ; beispielsweise besitzt das Natriumsalz der 3-(3-Carbamoyl-2,4,6-tribromphenyl)-propionsäure (A) die 6 000 bis 7 000-fache Süsskraft wie Saccharose (Rohrzucker) und übertrifft darin auch Saccharin-Natrium und Natriumcyclamat wie die Tabelle I zeigt.

(Siehe Tabelle Seite 5 f.)

Tabelle 1

Allgemeine geschmackliche Beurteilung und Süssungsgrad des Natriumsalzes von 3-(3-Carbamoyl-2,4,6-tribromphenyl)-propionsäure (A) im Vergleich zu Saccharose, Saccharin-Natrium und Natriumcyclamat.

| Stoff | Allgemeine geschmackliche Beurteilung* in den Konzentrationsstufen 1 - 8 | | | | | | | | Süssungsgrad* |
|---|---|---|---|---|---|---|---|---|---|
| | 1 | 2 | 3 | 4 | 5 | 6 | 7 | 8 | |
| Saccharose | - | - | - | - | - | - | - | - | 1 |
| Saccharin-Natrium | süss | süss | schwach süss | ganz schwach süss | - | - | - | - | 400 |
| Cyclamat | ganz schwach süss | o.G. | o.G. | o.G. | - | - | - | - | <100** |
| A | sehr, sehr süss | sehr süss | sehr süss | sehr süss | sehr süss | süss | süss | schwach süss | 6000 - 7000 |

* mittlere Aussage von 3 Geschmacksprüfungen ; o.G. = ohne Geschmack
** lt. Literatur : 30-70

Bestimmung der Süsskraft

a) Zur Bestimmung der Süsskraft wurde eine wässrige 5 %ige (g/v) Saccharose-Lösung mit wässrigen Lösungen des Natriumsalzes von 3-(3-Carbamoyl-2,4,6-tribromphenyl)-propionsäure (A) sowie von Saccharin-Natrium und Natriumcyclamat in bis zu acht Konzentrationsstufen durch Geschmacksprüfung verglichen. Der pH-Wert der 0,05 %igen Lösungen (Konzentrationsstufe 1), aus denen alle weiteren Lösungen durch Verdünnen mit Wasser (1 + 1) hergestellt wurden, betrug 5-7.

Folgende Konzentrationen entsprachen den 8 geprüften Konzentrationsstufen :

| Konzentrations-stufe-Nr. | Konzentration (mg/100 ml) | Konzentrationsverhältnis zur 5% Saccharose-Lösung |
|---|---|---|
| 1 | 50,0 | 1/100 |
| 2 | 25,0 | 1/200 |
| 3 | 12,5 | 1/400 |
| 4 | 6,25 | 1/800 |
| 5 | 3,125 | 1/1600 |
| 6 | 1,563 | 1/3200 |
| 7 | 0,781 | 1/6400 |
| 8 | 0,391 | 1/12800 |

Die Geschmacksuntersuchungen wurden von drei freiwilligen gesunden Versuchspersonen durchgeführt, wobei die Lösungen durch Buchstaben-Ziffern-Kombination kodiert und randomisiert waren, so dass der Untersucher nicht wissen konnte, welchen Stoff er verkostete. Auch die Bezugslössung (5 % Saccharose) blieb zum Zwecke der allgemeinen geschmacklichen Charakterisierung der Stoffe in den Randomisierungsplan zunächst eingeschlossen. Die Abschätzung des Süssungsgrades erfolgte dann in einer zweiten Prüfserie, bei der die Kodierung der Bezugslösung offengelegt wurde.

Die allgemeine geschmackliche Beurteilung der geprüften Stoffe und der Süssungsgrad werden in Tabelle 1 angegeben. Da die literaturbekannten (s. Hagers Handbuch der pharmazeutischen Praxis (4. Ausgabe) VII B (1977), Springer Verlag, S. 426) Süssungsgrade von Saccharin-Natrium (400) und von Natriumcyclamat (70) in blinder Geschmacksprüfung von allen drei Untersuchern sicher ermittelt wurden, können diese Ergebnisse als Grundlage für die Sicherheit der Prüfungsergebnisse der Testsubstanz gewertet werden.

b) Eine weitere sensorische Testreihe bestätigte die außerordentlich hohe Süßkraft der Verbindungen der Formel I im Vergleich zum Stand der Technik. In dieser Testreihe wurden die Erkennungsschwellenwerte ($C_{ts}$) der erfindungsgemäßen Verbindungen der Formel I

| | |
|---|---|
| 3-(3-Carbamoyl-2,4,6-tribromphenyl)-propionsäure | (A) |
| 3-(3-Carbamoyl-2,4,6-trijodphenyl)-propionsäure | (E) |
| 3-Carbamoyl-2,4,6-trijodphenoxy-essigsäure | (F) |
| 3-(3-Carbamoyl-2,4,6-trichlorphenyl)-propionsäure | (G) |

und der Verbindungen des Standes der Technik

Saccharin-Natrium
Cyclamat
Dulcin
Aspartame

ermittelt und mit dem Wert für Saccharose als natürlichem Süßungsmittel verglichen.

Diese sensorischen Untersuchungen wurden von 5 Personen durchgeführt ; alle Proben wurden von einer nicht an der Prüfung beteiligten Person verschlüsselt. Die Substanzen wurden in Leitungswasser gelöst, nötigenfalls wurde der pH-Wert mit NaOH auf 6-7 eingestellt. Verkostet wurden Proben von je 1 ml.

Die in einem Vorversuch ermittelten Konzentrations-Näherungswerte wurden im Hauptversuch in die Mitte einer Verdünnungsreihe gelegt. Alle Substanzlösungen wurden mit zwei Wasserproben verschlüsselt (Dreieckstest). Die Prüfer hatten bei fallender Konzentration jeweils anzugeben, welche der drei Proben sie als süß ansahen. Als Erkennungsschwellenwert wurde der niedrigste Konzentrationsbereich ($c_{ts}$) angegeben, in dem die Prüfer noch richtig urteilten.

Die Erkennungsschwellenwerte $c_{ts}$ [μMol/l] sowohl für die erfindungsgemäßen Verbindungen A, E, F und G als auch für Saccharin-Natrium, Cyclamat, Dulcin, Aspartame und Saccharose (Rohrzucker) als

Vergleichsverbindungen sind in Tabelle 2 zusammengestellt. Bildet man aus den $c_{ts}$-Werten der betreffenden Substanz und $c_{ts}$-Wert für Saccharose den Quotienten

$$f_{sac} = c_{ts,\ sac}/c_{ts,\ substanz}$$

erhält man eine Zahl f, die angibt, um wieviel höher die Süßkraft der geprüften Substanz im Vergleich zu Saccharose ist. Auch diese $f_{sac}$-Werte sind in Tabelle 2 zusammengestellt.

Tabelle 2

Erkennungsschwellenwerte ($c_{ts}$) einiger Süßstoffe

| Substanz | MG | (µMol/l) | $f_{sac,mol}(c_{ts})$ |
|---|---|---|---|
| G | 296,54 | 2,5 - 4,0 | 3385 |
| A | 451,89 | 0,6 - 1,4 | 11000 |
| E | 570,88 | 1,0 - 2,0 | 7333 |
| F | 584,92 | 15 - 25 | 550 |
| Saccharin-Natrium | 183,19 | 15 - 30 | 489 |
| Cyclamat | 201,22 | 1000 - 3000 | 5,5 |
| Dulcin | 180,2 | 15 - 30 | 489 |
| Aspartame | 294,3 | 20 - 60 | 275 |
| Saccharose | 342,3 | 10000 - 12000 | 1 |

Aus Tabelle 2 ist zu entnehmen, daß die erfindungsgemäßen Substanzen A, E, F und G eine größere Süßkraft (f ≥ 550) besitzen als das bisher bekannte stärkste künstliche Süßungsmittel Saccharin-Natrium (f = 489).

Die erfindungsgemässen Verbindungen der Formel Ia zeigen darüber hinaus eine ausgezeichnete allgemeine Verträglichkeit. Wie aus Tabelle 3 hervorgeht, ist nach intraperitonealer Injektion an Mäusen das Natriumsalz von 3-(3-Carbamoyl-2,4,6-tribromphenyl)-propionsäure (A) etwa gleich gut verträglich wie Saccharin-Natrium

Tabelle 3

| Prüfsubstanz | Dosis (g/kg KGW) | Befund (Zahl der toten Tiere/Zahl der eingesetzten Tiere) |
|---|---|---|
| A | 3,0 | 2/10 |
| | 6,0 | 6/10 |
| | 9,0 | 10/10 |
| Saccharin-Natrium | 3,0 | 0/10 |
| | 6,0 | 5/10 |
| | 10,0 | 9/10 |

Nach diesen Ergebnissen liegt die $LD_{50}$ nach einmaliger intraperitonealer Gabe von Saccharin-Natrium und von Verbindung A bei etwa 6,0 g/kg Körpergewicht. Damit entspricht die für Saccharin-Na gefundene $LD_{50}$ der früher von Taylor (Taylor, J. D., Richards, R. K. und Wiegand, R. G. : « Toxicological Studies with Sodium Cyclamate and Saccharin » Fd Cosmet Toxicol *6* : 313-327 (1968)) mitgeteilten $LD_{50}$ nach i.p. Gabe an Mäuse. Im Hinblick auf eine praktische Verwendung als Austauschstoff für Saccharin-Na ist diese hier nur grob einschätzbare gute Verträglichkeit von Substanz A als sehr günstig zu bewerten, da man voraussichtlich aufgrund der sehr hohen Süsskraft der erfindungsgemässen Verbindungen der Formel Ia mit einem Zehntel der Saccharin-Na Dosis auskommt.

Die Verbindungen der Formel Ia werden sowohl als Säuren als auch in Form ihrer Salze mit anorganischen Basen als Süßungsmittel verwendet.

Verbindungen der allgemeinen Formel Ia, worin Hal ein Jodatom darstellt, sind auch als Röntgenkontrastmittel geeignet.

Die Erfindung betrifft auch Süßungsmittel, gemäß Anspruch 1, die mindestens eine Verbindung der allgemeinen Formel I enthalten und in Form von Tabletten oder als Lösung zur Anwendung kommen. Die Herstellung der Süßungsmittel erfolgt nach Verfahren, die dem Fachmann bekannt sind. Zur Herstellung der Tabletten geht man zweckmäßigerweise so vor, daß man einen in Wasser löslichen Hilfsstoff und ein wasserlösliches Schmiermittel (Emulgator) mit mindestens einer Verbindung der Formel I mischt und in einer Tablettiermaschine Tabletten preßt. Das Gewicht einer Tablette soll dabei 40 mg bis 60 mg, vorzugsweise 50 mg betragen, wobei der Wirkstoffgehalt 5 mg bis 15 mg, vorzugsweise 10 mg und der Gehalt an Emulgator 0,2 mg bis 0,3 mg, vorzugsweise 0,25 mg betragen soll. Als wasserlösliche Hilfsstoffe kommen Substanzen infrage, die auch von Diabetikern vertragen werden wie beispielsweise Fructose, Lactose, Mannit oder Sorbit, wobei Fructose und Lactose bevorzugt sind. Als Schmiermittel (Emulgatoren) werden verwendet vorzugsweise nichtionogene Emulgatoren wie beispielsweise Polyoxyethylenpolyoxypropylen-Polymere, Polyoxyäthylenglykole, Ascorbylpalmitat, polyoxyethylierte Verbindungen wie Polyoxyethylenstearate oder Polyoxyethylenfettalkohole oder deren Äther, wobei Polyoxyethylenpolyoxypropylen-Polymeres mit dem Molgewicht 6 800 und Polyethylenglykole mit dem Molgewicht 6 000 bevorzugt sind.

Für die Herstellung einer Süßungsmittel-Lösung wird mindestens eine Verbindung der allgemeinen Formel I in destilliertem Wasser gelöst, wobei man der Lösung gegebenenfalls noch eine verdickend wirkende Substanz zusetzt, um die Handhabbarkeit und Dosierung zu erleichtern. Die Konzentration der Lösung an Verbindungen der Formel I beträgt 10 g bis 20 g pro Liter, wobei 15 g pro Liter bevorzugt ist. Als verdickend wirkende Substanzen kommen Gelatine, Hydroxypropylcellulose, Carboxymethylcellulose, wasserlösliche Celluloseäther infrage, von denen 0,1 % bis 0,8 % in der Lösung enthalten sind. Bevorzugt sind Gelatine und Hydroxypropylcellulose.

Die Erfindung betrifft auch ein Verfahren zur Herstellung von Verbindungen der allgemeinen Formel Ia, dadurch gekennzeichnet, daß man

a) eine Verbindung der allgemeinen Formel II

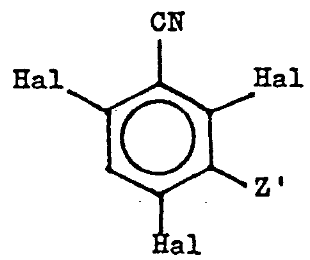

(II)

worin Hal ein Chlor-, Brom- oder Jodatom ist und Z' die Gruppe —COOH, —COOR[1] oder

$$-O_P-(CH_2)_K-O_L-(CH_2)_M-(\overset{X}{C}H)_N-\overset{Y}{C}H-COOR^1$$

bedeutet, wobei

$R^1$ ein Wasserstoffatom oder einen niederen Alkylrest mit 1 bis 4 Kohlenstoffatomen darstellt und P, K, L, M, N, X und Y die in Anspruch 1 angegebenen Bedeutungen haben, in an sich bekannter Weise durch Umsetzung mit Alkali hydrolysiert oder

b) zur Herstellung von Verbindungen der allgemeinen Formel Ia, worin Hal Chlor oder Brom ist und Z die Gruppe

$$-O_P-(CH_2)_K-O_L-(CH_2)_M-(\overset{X}{C}H)_N-\overset{Y}{C}H-COOH$$

darstellt, wobei P die Zahl 1 bedeutet, eine Verbindung der allgemeinen Formel III

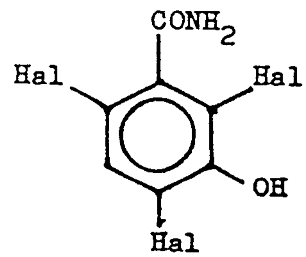

(III)

worin Hal ein Chlor- oder Bromatom darstellt, in an sich bekannter Weise in der Alkalimetallphenolatform umsetzt mit einer Verbindung der allgemeinen Formel IV

$$\mathrm{Hal'-(CH_2)_K-O_L-(CH_2)_M-(\overset{X}{\overset{|}{C}}H)_N-\overset{Y}{\overset{|}{C}}H-COOR^1} \quad \text{(IV)}$$

worin Hal' ein Chlor- oder Bromatom, $R^1$ einen niederen Alkylrest mit 1 bis 4 Kohlenstoffatomen darstellt und K, L, M, N, X und Y die in Anspruch 1 angegebenen Bedeutungen besitzen oder

Zur Herstellung von Verbindungen der allgemeinen Formel Ia, worin Hal Jod ist und Z die Gruppe

$$\mathrm{-O_P-(CH_2)_K-O_L-(CH_2)_M-(\overset{X}{\overset{|}{C}}H)_N-\overset{Y}{\overset{|}{C}}H-COOH}$$

darstellt, wobei P die Zahl 1 bedeutet, eine Verbindung der allgemeinen Formel III, worin Hal ein Jodatom darstellt, in an sich bekannter Weise in der Alkaliphenolatform umsetzt mit einer Verbindung der allgemeinen Formel IV, worin Hal' und $R^1$ die obengenannte Bedeutung haben

und, wenn L die Zahl 1 bedeutet,

K die Zahlen 2 bis 4,

M die Zahlen 0 ; 1 bis 3,

N die Zahl 0 oder 1,

X ein Wasserstoffatom und

Y ein Wasserstoffatom oder eine niedere Alkylgruppe mit 1 bis 4 Kohlenstoffatomen darstellt, oder

mit einer Verbindung der allgemeinen Formel IV umsetzt, worin Hal' und $R^1$ die obengenannte Bedeutung haben

und, wenn L die Zahl 0 bedeutet,

K, M und N die Zahl 0 darstellen und Y ein Wasserstoffatom ist, oder

mit einer Verbindung der allgemeinen Formel IV umsetzt, worin Hal' und $R^1$ die obengenannte Bedeutung haben

und, wenn L die Zahl 0 bedeutet,

K die Zahl 0,

M die Zahl 1 oder 2,

N die Zahl 0,

Y einen niederen Alkylrest mit 1 bis 4 Kohlenstoffatomen darstellt, oder

mit einer Verbindung der allgemeinen Formel IV umsetzt, worin Hal' und $R^1$ die obengenannte Bedeutung haben

und, wenn L die Zahl 0 bedeutet,

K die Zahlen 2 bis 4,

M die Zahlen 0 ; 1 bis 3

N die Zahl 1,

X ein Wasserstoffatom und

Y ein Wasserstoffatom oder einen niederen Alkylrest mit 1 bis 4 Kohlenstoffatomen bedeutet

und anschließend gegebenenfalls vorhandene Carbonsäurenieder-alkylester verseift und/oder durch Umsetzung mit anorganischen Basen Salze herstellt.

Die zur Herstellung der erfindungsgemäßen Verbindungen der allgemeinen Formel Ia erforderliche partielle Hydrolyse der Nitrilgruppe zur Carbamoylgruppe erfolgt nach dem Fachmann bekannten Verfahren. So kann man zum Beispiel die Nitrilgruppe dadurch in das Amid überführen, daß man die Ausgangsverbindung in konzentrierten Mineralsäuren wie Chlorwasserstoffsäure, Schwefelsäure oder

Phosphorsäure löst und die Hydrolyse bei Temperaturen von ca. Raumtemperatur bis 100 °C durchführt, vorzugsweise bei 50 °C bis 100 °C. Man kann die Nitrilgruppe aber auch in alkalischem Milieu partiell hydrolysieren, indem man die Ausgangsverbindung in wäßrigem Alkalihydroxid löst oder suspendiert und bei einer Temperatur von ca. Raumtemperatur bis 100 °C, vorzugsweise bei 40 °C bis 80 °C hydrolysiert.

Die für die Verfahrensvariante a) benötigten Ausgangsverbindungen der allgemeinen Formel II sind zum Teil bekannt oder können nach dem Fachmann bekannten Verfahren hergestellt werden. So werden beispielsweise Verbindungen der Formel II, worin Hal ein Jodatom und Z' entweder —COOH oder die Gruppe

$$-O_P-(CH_2)_K-O_L-(CH_2)_M-(\overset{X}{\overset{|}{CH}})_N-\overset{Y}{\overset{|}{CH}}-COOH$$

bedeutet und P und L die Zahl 0 darstellen, in der Deutschen Offenlegungsschrift 28 31 496 zusammen mit Verfahren zu ihrer Herstellung beschrieben.

Zur Herstellung von Verbindungen der Formel II, worin Hal ein Brom- oder Chloratom bedeutet und Z' entweder —COOH oder die Gruppe

$$-O_P-(CH_2)_K-O_L-(CH_2)_M-(\overset{X}{\overset{|}{CH}})_N-\overset{Y}{\overset{|}{CH}}-COOH$$

bedeutet und P und L die Zahl 0 darstellt, kann man beispielsweise ein Anilinderivat der allgemeinen Formel V mit einem Halogenierungsmittel wie beispielsweise Sulfurylchlorid in einem geeigneten Lösungsmittel wie beispielsweise Benzol, Toluol oder Äthylenchlorid oder mit molekularem Halogen in einem geeigneten Lösungsmittel wie beispielsweise Eisessig oder Äthylenchlorid zum trihalogenierten Anilin der allgemeinen Formel VI umsetzen

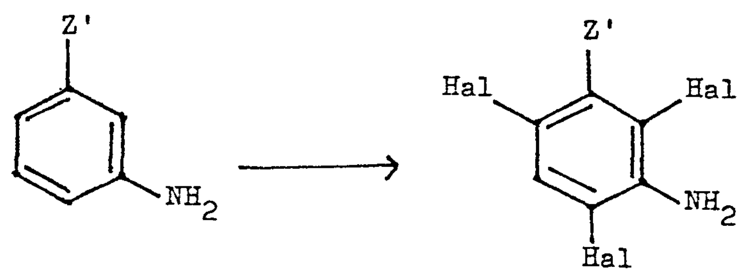

(V) (VI)

wobei Hal ein Brom- oder Chloratom bedeutet und
Z' entweder —COOH oder die Gruppe

$$-O_P-(CH_2)_K-O_L-(CH_2)_M-(\overset{X}{\overset{|}{CH}})_N-\overset{Y}{\overset{|}{CH}}-COOH$$

darstellt und P und L die Zahl 0 bedeuten.

Die so erhaltenen trihalogenierten Anilinderivate der Formel VI werden nach dem in der Deutschen Offenlegungsschrift Nr. 28 31 496 beschriebenen Verfahren in einer Sandmeyer-Reaktion durch Diazotierung und anschließende Umsetzung mit Cyaniden in die Benzonitrile der allgemeinen Formel II überführt, worin Z' entweder —COOH oder die Gruppe

$$-O_P-(CH_2)_K-O_L-(CH_2)_M-(\overset{X}{\overset{|}{CH}})_N-\overset{Y}{\overset{|}{CH}}-COOH$$

bedeutet und P und L die Zahl 0 darstellen.

Die nachfolgenden Herstellungsvorschriften für 3-(3-Cyano-2,4,6-trichlorphenyl)-propionsäure und für die entsprechende 2,4,6-Tribromverbindung sollen die einzelnen Reaktionsschritte erläutern :

3-(3-Cyano-2,4,6-trichlorphenyl)-propionsäure :

30 g 3-(3-Aminophenyl)-propionsäure werden in 2 l Benzol suspendiert. Man tropft 220 ml Sulfurylchlorid zu und erhitzt den Ansatz 90 Minuten zum Rückfluss. Man filtriert heiss von wenig ungelöstem Ausgangsmaterial ab und engt das Filtrat zur Trockne ein. Der Rückstand wird mit 900 ml Wasser versetzt und 1 Stunde auf dem Dampfbad unter Rühren erhitzt. Dann setzt man portionsweise 366 ml einer 25 %igen Sodalösung zu und erhitzt erneut 30 Minuten unter Rühren auf dem Dampfbad. Nach Klären der Lösung über Aktivkohle bringt man unter Rühren und Kühlen durch Zugabe von konzentrierter Salzsäure auf pH 2. Nach mehrstündigem Rühren im Eisbad wird der Niederschlag abgesaugt, mit Wasser gewaschen und bei 50 °C getrocknet. Man erhält 33 g (68 % der Theorie) 3-(3-Amino-2,4,6-trichlorphenyl)-propionsäure als weisses Pulver vom Fp. : 145 °C.

7 g Natriumnitrit werden unter Rühren bei + 5 °C in 84 ml konzentrierter Schwefelsäure eingetragen. Man lässt 10 Minuten nachrühren und erhitzt anschliessend bis 70 °C zur klaren Lösung. Nach Abkühlen auf 5 °C gibt man unter Eisbadkühlung 42 ml Eisessig zu. Dann werden zwischen 0 °C und + 5 °C portionsweise 21,5 g 3-63-Amino-2,4,6-trichlorphenyl)-propionsäure eingetragen und der Ansatz 2 Stunden bei 5 °C nachgerührt. Man giesst auf 200 g Eis auf und trägt anschliessend unter Rühren in eine Lösung von 35,4 g Kupfer-I-cyanid und 66,8 g Kaliumcyanid in 900 ml halbkonzentriertem Ammoniak ein, wobei Stickstoffentwicklung eintritt. Nach Rühren über Nacht bei Raumtemperatur werden 800 ml Essigester zugesetzt und der Ansatz durch tropfenweise Zugabe von konzentrierter Salsäure auf $p_H$ 1 gebracht. Nacht Absaugen der ausgeschiedenen anorganischen Salze wird die Essigesterphase abgetrennt, zweimal mit je 300 ml Wasser gewaschen und nach Trocknen über Natriumsulfat zur Trockne eingeengt. Nach Umkristallisieren aus der siebenfachen Menge Toluol erhält man 16 g (72 % der Theorie) 3-(3-Cyano-2,4,6-trichlorphenyl)-propionsäure als weisses Pulver vom Fp. : 127-129 °C

In analoger Weise werden hergestellt :

3-Cyano-2,4,6-trichlorphenylessigsäure (Fp. : 182-184 °C) mit einer Gesamtausbeute von 49 % der Theorie aus 3-Aminophenyl-essigsäure (Fp. : 152 °C) über die Stufe der 3-Amino-2,4,6-trichlor-phenylessigsäure (Fp. : 206 °C)

und

3-Cyano-2,4,6-trichlorbenzoesäure (Fp. : 138-142 °C) mit einer Gesamtausbeute von 42 % der Theorie aus 3-Aminobenzoesäure (Fp. : 178 °C) über die Stufe der 3-Amino-2,4,6-trichlorbenzoesäure (Fp. : 159-161 °C).

3-(3-Cyano-2,4,6-tribromphenyl)-propionsäure :

32,7 g 3-(3-Aminophenyl)-propionsäure werden in 1 l Wassersuspendiert und durch Zugabe von konzentriertem Ammoniumhydroxid gelöst. Man gibt 160 ml Eisessig zu und tropft anschliessend innerhalb 2 Stunden eine Lösung von 36 ml Brom in 160 ml Eisessig bei Raumtemperatur unter intensivem Rühren zu. Man lässt 2 Stunden nachrühren, saugt den Niederschlag ab, wäscht ihn mit Wasser, trocknet ihn bei 50 °C und kristallisiert ihn aus der vierfachen Menge Essigester unter Aktivkohlebehandlung um. Man erhält 63 g (78 % der Theorie) 3-(3-Amino-2,4,6-tribromphenyl)-propionsäure als weisses Pulver vom Fp. : 195-197 °C.

3,5 g Natriumnitrit werden bei 5 °C unter Rühren in 42 ml konzentrierter Schwefelsäure eingetragen. Man lässt 10 Minuten nachrühren und erhitzt anschliessend bis 70 °C zur klaren Lösung. Nach Abkühlen auf 5 °C gibt man unter Kühlung im Eisbad 21 ml Eisessig zu. Dann werden zwischen 0° und 5 °C portionsweise 16 g 3-(3-Amino-2,4,6-tribromphenyl)-propionsäure eingetragen und der Ansatz 2 Stunden bei 5 °C nachgerührt. Man giesst auf 200 g Eis auf und trägt anschliessend unter Rühren in eine Lösung von 18 g Kupfer-I-cyanid und 33,5 g Kaliumcyanid in 450 ml halbkonzentriertem Ammoniak ein, wobei Stickstoffentwicklung eintritt. Man sättigt die Lösung mit Kochsalz ab und lässt über Nacht unter Wasserkühlung rühren. Das ausgeschiedene Ammonsalz wird abgesaugt und feucht in 200 ml Wasser gelöst. Die Lösung wird nach Behandlung mit Aktivkohle durch Zugabe von konzentrierter Salzsäure auf pH 1 gebracht. Nach mehrstündigem Rühren im Eisbad wird der Niederschlag abgesaugt, mit Wasser gewaschen und bei 50 °C getrocknet. Man erhält 13 g (83 % der Theorie) 3-(3-Cyano-2,4,6-tribromphenyl)-propionsäure als weisses Pulver vom Fp. : 164-166 °C.

In analoger Weise werden hergestellt :

3-Cyano-2,4,6-tribromphenylessigsäure (Fp. : 233 °C) mit einer Ausbeute von 62 % der Theorie aus 3-Aminophenylessigsäure (Fp. : 152 °C) über die Stufe der 3-Amino-2,4,6-tribromphenylessigsäure (Fp. : 229-231 °C)

und

3-Cyano-2,4,6-tribrombenzoesäure (Fp. : 168 °C) mit einer Ausbeute von 75 % der Theorie aus 3-Aminobenzoesäure (Fp. : 178 °C) über die Stufe der 3-Amino-2,4,6-tribrombenzoesäure (Fp. : 180 °C).

Zur Herstellung von Verbindungen der allgemeinen Formel Ia, worin P die Zahl 1 bedeutet, nach der Verfahrensvariante a)

geht man zweckmäßigerweise von den entsprechenden 2,4,6-Trihalogen-3-hydroxy-benzonitrilen aus, die als Tribrom- und Trichlorverbindungen bekannt sind. Das bisher unbekannte 2,4,6-Trijod-3-hydroxybenzonitril läßt sich durch Jodierung von 3-Hydroxybenzonitril wie folgt herstellen :

Eine Lösung von 11,9 g (100 mMol) 3-Hydroxybenzonitril in 130 ml Eisessig wird innerhalb 2 Stunden mit einer Lösung von 53 g (327 mMol) Chlorjodid in 80 ml Eisessig versetzt. Man tropft nach 30 Minuten 400 ml Wasser zu und läßt den Ansatz 24 Stunden bei 35 °C nachrühren. Anschließend wird auf Raumtemperatur abgekühlt und das überschüssige Chlorjodid durch Zugabe von festem Natriumbisulfit zersetzt. Das abgesaugte Jodierungsprodukt wird mit Natriumbisulfitlösung, danach mit Wasser neutral gewaschen und getrocknet. Man erhält 31 g (62 % der Theorie) 3-Hydroxy-2,4,6-trijod-benzonitril als cremefarbenes Pulver vom Schmelzpunkt 242-245 °C (Zersetzung).

Die 2,4,6-Trihalogen-3-hydroxybenzonitrile werden dann mit der gewünschten Halogenverbindung der allgemeinen Formel IV alkyliert nach Verfahren, die dem Fachmann bekannt sind.

Das folgende Beispiel soll die Alkylierung näher erläutern :

3-Cyano-2,4,6-trichlorphenoxyessigsäure-methylester

22,5 g (0,1 Mol) 3-Hydroxy-2,4,6-trichlorbenzonitril werden in 240 ml Methanol gelöst unter Zugabe von 2,3 g (0,1 Mol) Natrium. Zur Suspension des ausgeschiedenen Natriumsalzes gibt man 12,15 ml (0,11 Mol) Bromessigsäureäthylester und erhitzt solange zum Rückfluß, bis Lösung eingetreten ist. Nach Filtration über Aktivkohle wird die Lösung abgekühlt und nach mehrstündigem Rühren im Eisbad das ausgeschiedene Kristallisat abgesaugt, mit wenig eiskaltem Methanol gewaschen und bei 50 °C getrocknet. Man erhält 19 g (64,5 % der Theorie) 3-Cyano-2,4,6-trichlorphenoxyessigsäuremethylester als weißes Pulver vom Schmelzpunkt 110-112 °C.

Die für die Alkylierung sowohl der oben beschriebenen 2,4,6-Trihalogen-3-hydroxy-benzonitrile als auch der Verbindungen der allgemeinen Formel III der Verfahrensvariante b), die nachfolgend beschrieben wird, benötigten Halogenverbindungen der allgemeinen Formel IV sind zum größten Teil bekannt oder können nach bekannten Verfahren, wie sie beispielsweise in der europäischen Patentschrift 0001740 beschrieben sind, hergestellt werden.

Stellt man die erfindungsgemäßen Verbindungen der allgemeinen Formel I a, worin P die Zahl I bedeutet, nach der Verfahrensvariante b) her, so erfolgt dies mit Methoden, wie sie dem Fachmann bekannt sind. So kann man beispielsweise das Phenol der allgemeinen Formel III, gelöst in einem ein- oder mehrwertigen Alkohol wie Methanol, Äthanol, Glykol, Glycerin, aber auch Diäthylenglykol oder Dimethylformamid, mit der äquivalenten Menge Alkalialkoholat in das Alkaliphenolat überführen und durch Erhitzen gegebenenfalls bis zum Sieden mit der etwas mehr als der äquivalenten Menge Alkylhalogenid der allgemeinen Formel IV in den Phenoläther überführen, wie es dem Fachmann bekannt ist. Vorzugsweise verwendet man bei der Durchführung der Verfahrensvariante b) Natriumalkoholat als Base, Methanol oder Äthanol als Lösungsmittel und als Alkylhalogenid der Formel IV das entsprechende Chlorid oder Bromid.

Es ist aber auch möglich, das Phenol der Formel III in einem geeigneten Lösungsmittel wie beispielsweise Aceton, mit einem Überschuss Alkalicarbonat, vorzugsweise Kaliumcarbonat und dem gewünschten Alkylhalogenid der allgemeinen Formel IV, das mit ca. 10 % Überschuss angewendet wird, in der Siedehitze umzusetzen, wobei ein Zusatz von Alkalijodid die Umsetzung erleichtert. Werden höhere Umsetzungstemperaturen erforderlich, kann man höhersiedende Ketone wie Methyläthylketon oder Cyclohexanon anwenden. Als Alkylhalogenide der allgemeinen Formel IV werden vorzugsweise das Chlorid oder Bromid verwendet.

Die für die Durchführung der Verfahrensvariante b) erforderlichen Ausgangsstoffe der allgemeinen Formel III :

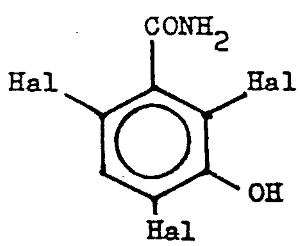

(III)

sind mit Hal = Jod bekannt (G. Tilly, Chim. Ther. 2 [1], 57-65 [1967]) oder können nach Verfahren, die dem Fachmann bekannt sind, aus 3-Hydroxybenzamid durch Halogenierung hergestellt werden gemäß den nachfolgenden Vorschriften :

3-Hydroxy-2,4,6-trichlorbenzamid

25 g (180 mMol) 3-Hydroxybenzamid werden in 500 ml Eisessig gelöst. Dann leitet man bei 35 °C in die Lösung 26 g Chlor ein und läßt 4 Stunden bei 60 °C nachrühren. Nach dem Einengen im Vakuum zur Trockne versetzt man den Rückstand mit 150 ml Wasser, läßt mehrere Stunden im Eisbad nachrühren

und saugt den Niederschlag ab. Nach Waschen mit Wasser und Trocknen erhält man 34 g (80 % der Theorie) 3-Hydroxy-2,4,6-trichlorbenzamid als weißes Pulver vom F. 204-206 °C.

3-Hydroxy-2,4,6-tribrombenzamid

40 g (0,29 Mol) 3-Hydroxybenzamid werden in 1,5 l Wasser suspendiert und durch Zugabe von konzentriertem Ammoniak in Lösung gebracht. Man bringt die Lösung anschließend durch Zugabe von Eisessig auf pH 5 und tropft innerhalb zwei Stunden bei Raumtemperatur eine Lösung von 79,9 g (1Mol) Brom in 200 ml Eisessig zu. Man läßt zwei Stunden nachrühren, saugt den gebildeten Niederschlag ab, wäscht ihn mit 500 ml einer 10 %igen Natriumbisulfitlösung, wäscht ihn mit Wasser und trocknet ihn bei 60 °C. Man erhält 103 g (95 % der Theorie) 3-Hydroxy-2,4,6-tribrombenzamid als gelblich-weißes Pulver vom F. 225-226 °C (Zersetzung).

Ist es im Verlaufe des erfindungsgemäßen Verfahrens gegebenenfalls erforderlich, entstandendene Carbonsäurenieder-Alkylester zu verseifen, so führt man das nach Verfahren aus, die dem Fachmann bekannt sind, So ist es möglich, den Ester in Wasser zu suspendieren, einen Überschuß an Alkalihydroxid zuzugeben und bei einer Temperatur von 20 °C bis 80 °C, vorzugsweise bei 50 °C bis 70 °C die Verseifung durchzuführen. Anschließend wird die Lösung durch Filtration, vorzugsweise über Aktivkohle, gereinigt und bis zur sauren Reaktion Mineralsäure zugegegeben, worauf die Säure der allgemeinen Formel Ia isoliert werden kann. Zur Herstellung der Alkalisalze wird die Säure der allgemeinen Formel Ia in einem geeigneten Lösungsmittel, beispielsweise in einem Gemisch von Äthanol oder Methanol mit Wasser suspendiert und mit der äquivalenten Menge verdünntem Alkalihydroxid, vorzugsweise Natrium- oder Kaliumhydroxid, in das Alkalisalz überführt, die Lösung durch Filtration gereinigt und das Salz isoliert.

Die nachfolgenden Ausführungsbeispiele sollen die Erfindung näher erläutern :

Beispiel 1

3-(3-Carbamoyl-2,4,6-trichlorphenyl)-propionsäure :

16 g 3-(3-Cyano-2,4,6-trichlorphenyl)-propionsäure werden in einer Lösung von 5 g Ätznatron in 80 ml Wasser gelöst. Man hält den Ansatz 3 Stunden auf 60 °C, filtriert über Aktivkohle und bringt das Filträt unter Kühlung durch Zugabe von konzentrierter Salzsäure unter Rühren auf pH 1. Nach mehrstündigem Rühren wird der Niederschlag abgesaugt, mit Wasser gewaschen und bei 50 °C getrocknet. Nach Umkristallisieren aus der fünffachen Menge Toluol erhält man 15,5 g (90 % der Theorie) 3-(3-Carbamoyl-2,4,6-trichlorphenyl)-propionsäure als weisses Pulver vom Fp. : 196-198 °C.

Beispiel 2

3-Carbamoyl-2,4,6-trichlorphenylessigsäure :

Wie im Beispiel 1 beschrieben, erhält man aus 3-Cyano-2,4,6-trichlorphenylessigsäure mit einer Ausbeute von 88 % der Theorie 3-Carbamoyl-2,4,6-trichlorphenylessigsäure ; Fp. : 211 °C.

Beispiel 3

2,4,6-Trichlorisophthalamsäure :

Wie im Beispiel 1 beschrieben, erhält man aus 3-Cyano-2,4,6-trichlorbenzoesäure mit einer Ausbeute von 88 % der Theorie 2,4,6-Trichlorisophthalamsäure ; Fp. : 280 °C.

Beispiel 4

3-(3-Carbamoyl-2,4,6-tribromphenyl)-propionsäure :

A. Alkalische Verseifung :

13 g 3-(3-Cyano-2,4,6-tribromphenyl)-propionsäure werden in eine Lösung von 3,2 g Ätznatron in 64 ml Wasser eingetragen. Dann rührt man die Lösung 3 Stunden bei 60 °C, filtriert zur Entfärbung mehrfach über Aktivkohle und bringt anschliessend das Filtrat durch Zugabe von konzentrierter Salzsäure auf pH 1. Der Niederschlag wird abgesaugt, mit Wasser gewaschen und bei 50 °C getrocknet. Man erhält 16 g (95 % der Theorie) 3-(3-Carbamoyl-2,4,6-tribromphenyl)-propionsäure als weisses Pulver vom Fp. : 197 °C.

B. Saure Hydrolyse :

13 g 3-(3-Cyano-2,4,6-tribromphenyl)-propionsäure werden in 50 ml konzentrierter Schwefelsäure

suspendiert und zunächst 30 Minuten auf 60 °C, danach 2 Stunden auf 95 °C erwärmt. Danach wird die Lösung auf 300 g Eis gegeben und 3 Stunden im Eisbad nachgerührt. Der ausgeschiedene Niederschlag wird abgesaugt, mit Eiswasser gewaschen und bei 50 °C im Vakuum getrocknet. Man erhält 14 g 3-(3-Carbamoyl-2,4,6-tribromphenyl)-propionsäure mit einer Ausbeute von 90 % der Theorie ; Fp. : 197 °C.

Beispiel 5

3-Carbamoyl-2,4,6-tribromphenylessigsäure :

Wie in Beispiel 4 beschrieben, erhält man aus 3-Cyano-2,4,6-tribromphenylessigsäure mit einer Ausbeute von 90 % der Theorie 3-Carbamoyl-2,4,6-tribromphenylessigsäure ; Fp. : 245 °C.

Beispiel 6

2,4,6-Tribromisophthalamsäure :

Wie in Beispiel 4 beschrieben, erhält man aus 3-Cyano-2,4,6-tribrombenzoesäure mit einer Ausbeute von 92 % der Theorie 2,4,6-Tribromisophthalamsäure ; Fp. : > 280 °C.

Beispiel 7

3-(3-Carbamoyl-2,4,6-trijodphenyl)-propionsäure :

30 g 3-(3-Cyano-2,4,6-trijodphenyl)-propionsäure werden in eine Lösung von 8 g Ätznatron in 100 ml Wasser eingetragen. Dann hält man die Lösung 3 Stunden bei 60 °C, entfärbt sie durch mehrfache Behandlung mit Aktivkohle und bringt sie durch Zugabe von konzentrierter Salzsäure auf pH 1. Nach mehrstündigem Rühren im Eisbad wird der Niederschlag abgesaugt, mit Wasser gewaschen und bei 50 °C getrocknet. Man erhält 29 g (94 % der Theorie) 3-(3-Carbamoyl-2,4,6-trijodphenyl)-propionsäure als weisses Pulver ; Fp. : > 280 °C.

In analoger Weise werden aus den entsprechenden Nitrilen erhalten :
2,4,6-Trijodisophthalamsäure,
3-(3-Carbamoyl-2,4,6-trijodphenyl)-2-äthyl-propionsäure,
3-(3-Carbamoyl-2,4,6-trijodphenyl)-acrylsäure,
3-(3-Carbamoyl-2,4,6-trijodphenyl)-2-n-amyloxy-propionsäure,
5-(3-Carbamoyl-2,4,6-trijodphenyl)-pentansäure.

Beispiel 8

3-Carbamoyl-2,4,6-tribromphenoxyessigsäure

35,5 g (95 mMol) 3-Hydroxy-2,4,6-tribrombenzamid werden in 150 ml Methanol suspendiert und durch Zugabe von 2,18 g (95 mMol) Natrium gelöst. Man setzt 17,5 g (104 mMol) Bromessigsäureäthylester zu und hält drei Stunden am Rückfluß. Nach mehrstündigem Rühren wird das Kristallisat abgesaugt, mit wenig eiskaltem Methanol gewaschen und bei 60 °C getrocknet. Man erhält 37,2 g (88 % d. Theorie) 3-Carbamoyl-2,4,6-tribromphenoxyessigsäuremethylester vom F. 231-233 °C. 30 g (67 mMol) 3-Carbamoyl-2,4,6-tribromphenoxyessigsäuremethylester werden in 300 ml Wasser suspendiert, die Suspension auf 60 °C erwärmt und der Ester durch Zutropfen von 25 ml konzentrierter Natronlauge verseift. Man filtriert die Lösung über Aktivkohle und bringt sie durch tropfenweise Zugabe von konzentrierter Salzsäure unter Eisbadkühlung auf pH 1. Nach mehrstündigem Nachrühren saugt man den gebildeten Niederschlag ab, wäscht ihn mit Wasser und trocknet ihn bei 60 °C. Man erhält 28 g (97 % der Theorie) 3-Carbamoyl-2,4,6-tribromphenoxyessigsäure als weißes Pulver vom F. : 271-272 °C (Zersetzung).

Beispiel 9

4-(3-Carbamoyl-2,4,6-tribromphenoxy)-buttersäure

25 g (66,9 mMol) 3-Hydroxy-2,4,6-tribrombenzamid werden in 100 ml Methanol suspendiert und durch Zugabe von 1,54 g (66,9 mMol) Natrium in Lösung gebracht. Nach Zugabe von 14 g (73 mMol) 4-Brombuttersäureäthylester hält man den Ansatz 70 Stunden am Rückfluß, engt dann die Lösung zur Trockne ein und kocht den Rückstand mit 250 ml Äther aus. Nach mehrstündigem Rühren im Eisbad wird der Niederschlag abgesaugt, mit Äther gewaschen und bei Raumtemperatur getrocknet. Man erhält 19 g (58 % der Theorie) 4-(3-Carbamoyl-2,4,6-tribromphenoxy) buttersäureäthylester als weißes Pulver vom F. 138-140 °C.

18 g (36,9 mMol) 4-(3-Carbamoyl-2,4,6-tribromphenoxy) buttersäure-athylester werden in 180 ml

Wasser suspendiert und nach Zugabe von 10 ml konzentrierter Natronlauge bei 60 °C innerhalb drei Stunden verseift. Man filtriert die Lösung über Aktivkohle und bringt sie durch tropfenweise Zugabe von konzentrierter Salzsäure auf pH 1. Nach mehrstündigem Rühren im Eisbad wird der gebildete Niederschlag abgesaugt, mit Wasser gewaschen und bei 60 °C getrocknet. Man erhält 15 g (88 % der Theorie) 4-(3-Carbamoyl-2,4,6-tribromphenoxy) buttersäure als weißes Pulver vom F. 193-194 °C.

Beispiel 10

5-(3-Carbamoyl-2,4,6-trichlor-phenoxy)-3-oxa-pentansäure-Natriumsalz

2,3 g (10 mMol) Natrium werden in 200 ml Äthanol gelöst. Man gibt dann 24 g (10 mMol) 3-Hydroxy-2,4,6-trichlorbenzamid, 16,7 g (10 mMol) 5-Chlor-3-oxapentansäureäthylester und 0,3 g Kaliumjodid zu und hält den Ansatz 24 Stunden am Rückfluß. Nach Zugabe von 1 Liter Wasser läßt man 30 Minuten im Eisbad rühren, saugt den ausgeschiedenen Niederschlag ab und wäscht ihn mit Wasser.

Anschließend wird der feuchte 5-(3-Carbamoyl-2,4,6-trichlorphenoxy)-3-oxapentansäureäthylester in 250 ml Wasser suspendiert und nach Zugabe von 40 ml halbkonzentrierter Natronlauge bei 60 °C innerhalb drei Stunden verseift. Man filtriert die Lösung über Aktivkohle, kühlt sie auf Raumtemperatur ab und bringt sie durch Zugabe von konzentrierter Salzsäure auf pH 1. Nach mehrstündigem Rühren im Eisbad wird die ausgeschiedene 5-(3-Carbamoyl-2,4,6-trichlor-phenoxy)-3-oxapentansäure abgesaugt, mit Wasser neutral gewaschen und anschließend in 300 ml eines Gemisches aus gleichen Teilen Äthanol und Wasser nach Neutralisation durch Zugabe von 2n-Natronlauge gelöst. Die Lösung wird mit Aktivkohle behandelt und anschließend im Vakuum zur Trockne eingeengt. Man erhält 29,8 g (82 % der Theorie) Natriumsalz der 5-(3-Carbamoyl-2,4,6-trichlor-phenoxy)-3-oxa-pentansäure.

Beispiel 11

3-Carbamoyl-2,4,6-trichlor-phenoxy-essigsäure

16 g (66,9 mMol) 3-Hydroxy-2,4,6-trichlorbenzamid werden in 100 ml Methanol suspendiert und durch Zugabe von 1,54 g (66,9 mMol) Natrium in Lösung gebracht. Nach Zugabe von 16,8 g (66,9 mMol) Bromessigsäureäthylester hält man den Ansatz solange am Rückfluß, bis die Umsetzung vollständig ist (DC-Kontrolle). Dann engt man im Vakuum zur Trockne ein und suspendiert den erhaltenen 3-Carbamoyl-2,4,6-trichlorphenoxy-essigsäure-methylester in 100 ml Wasser. Man setzt 20 ml konzentrierte Natronlauge zu und hält zur Verseifung 3 Stunden bei 60 °C. Man filtriert die Lösung über Aktivkohle, bringt sie durch Zugabe von konzentrierter Salzsäure auf pH 2 und saugt den Niederschlag nach mehrstündigem Rühren im Eisbad ab. Nach Waschen mit Wasser und Trocknen erhält man 16,8 g (84 % der Theorie) 3-Carbamoyl-2,4,6-trichlor-phenoxy-essigsäure als weißes Pulver vom F. 261-262 °C.

Beispiel 12

4-(3-Carbamoyl-2,4,6-trichlor-phenoxy)-buttersäure

16 g (66,9 mMol) 3-Hydroxy-2,4,6-trichlorbenzamid werden in 100 ml Methanol suspendiert und durch Zugabe von 1,54 g (66,9 mMol) Natrium in Lösung gebracht. Nach Zugabe von 14 g (73 mMol) 4-Brombuttersäure-äthylester hält man den Ansatz 70 Stunden am Rückfluß, engt dann die Lösung zur Trockne ein und kocht den Rückstand mit Äther aus. Nach mehrstündigem Rühren im Eisbad saugt man das Kristallisat ab, wäscht es mit eiskaltem Äther und trocknet es bei Raumtemperatur. Man erhält 13,4 g (59 % der Theorie) 4-(3-Carbamoyl-2,4,6-trichlor-phenoxy)-buttersäure-methylester als weißes Pulver vom F. 117-118 °C. Man suspendiert die Verbindung anschließend in 150 ml Wasser und verseift sie nach Zugabe von 8 ml konzentrierte Natronlauge innerhalb 3 Stunden bei 60 °C. Man filtriert die Lösung über Aktivkohle und bringt sie durch Zugabe von konzentrierter Salzsäure auf pH 2. Nach mehrstündigem Rühren im Eisbad saugt man den Niederschlag ab, wäscht ihn mit Wasser und trocknet ihn bei 50 °C. Man erhält 11,4 g (89 % der Theorie) 4-(3-Carbamoyl-2,4,6-trichlor-phenoxy) buttersäure als weißes Pulver vom F. : 165-166 °C.

Beispiel 13

3-Carbamoyl-2,4,6-trijod-phenoxyessigsäure

0,436 g (19 mMol) Natrium werden in 20 ml Äthanol gelöst. Dann gibt man 9,78 g (19 mMol) 3-Hydroxy-2,4,6-trijod-benzamid und anschließend 3,5 g (21 mMol) Bromessigsäureäthylester zu. Man erhitzt eine Stunde zum Rückfluß und saugt nach mehrstündigem Rühren im Eisbad den gebildeten 3-Carbamoyl-2,4,6-trijod-phenoxy-essigsäureäthylester ab, wäscht ihn mit wenig eiskaltem Äthanol und suspendiert ihn in 80 ml Wasser. Man bringt durch Zugabe von konzentrierter Natronlauge auf pH 12, läßt 30 Minuten bei 60 °C rühren, behandelt die Lösung mit 1 g Aktivkohle, filtriert und bringt durch Zutropfen

von verdünnter Salzsäure auf pH 1. Nach mehrstündigem Rühren im Eisbad wird der gebildete Niederschlag abgesaugt, mit Wasser gewaschen und getrocknet. Man erhält 10,2 g (89 % der Theorie) 3-Carbamoyl-2,4,6-trijod-phenoxyessigsäure als weißes Pulver vom F. 211-213 °C (Zersetzung).

Beispiel 14

3-Carbamoyl-2,4,6-trijod-phenoxyessigsäure

27,3 g (49 mMol) 3-Cyano-2,4,6-trijod-phenoxyessigsäure werden in 100 ml 2n Natronlauge 3 Stunden bei 50 °C gerührt. die erhaltene alkalische Lösung wird zur Reinigung und Entfärbung über Aktivkohle filtriert und anschließend durch Zugabe von 2n Salzsäure auf pH = 2 gebracht. Nach mehrstündigem Rühren im Eisbad erhält man 23,9 g (85 % der Theorie) 3-Carbamoyl-2,4,6-trijod-phenoxyessigsäure als weißes Pulver vom F. 211-213 °C (Zersetzung).

Beispiel 15

3-Carbamoyl-2,4,6-trichlor-phenoxy-essigsäure

29,4 g (0,1 Mol) 3-Cyano-2,4,6-trichlor-phenoxy-essigsäuremethylester werden in 300 ml Wasser suspendiert. Nach Zugabe von 35 ml konzentrierter Natronlauge hält man den Ansatz solange bei 60 °C, bis klare Lösung eingetreten ist. Nach Filtration über Aktivkohle wird die Lösung abgekühlt und durch tropfenweise Zugabe von halbkonzentrierter Salzsäure auf pH 2 gebracht. Man läßt zwei Stunden im Eisbad nachrühren, saugt den gebildeten Niederschlag ab, wäscht ihn mit Wasser und trocknet ihn bei 50 °C. Man erhält 25,4 g (85 % der Theorie) 3-Carbamoyl-2,4,6-trichlor-phenoxy-essigsäure als weißes Pulver vom Schmelzpunkt 261-262 °C.

Beispiel 16

3-(3-Carbamoyl-2,4,6-tribromphenyl)-2-methoxy-propionsäure

50 g (223 mMol) 3-(3-Nitrophenyl)-2-methoxy-acrylsäure werden in 785 ml Wasser suspendiert und durch Zugabe von 30 ml konzentriertem Ammoniak in Lösung gebracht. Nach Zugabe von 3 g Raney-Nickel wird bei 80 bar bis zur Beendigung der Wasserstoffaufnahme hydriert. Nach Filtration versetzt man die Lösung mit 325 g Kochsalz und extrahiert sie erschöpfend mit Tetrahydrofuran. Die vereinigten Tetrahydrofuranextrakte werden über Natriumsulfat getrocknet und nach Filtration über Aktivkohle eingeengt. Man erhält 38,6 g (88 % der Theorie) 3-(3-Aminophenyl)-2-methoxypropionsäure, die nach Zugabe von 110 ml 2n-Ammonialklösung in einem Liter Wasser gelöst wird. Diese Lösung wird zuerst mit 160 ml Eisessig, darauf innerhalb 2 Stunden bei Raumtemperatur tropfenweise mit einer Mischung von 36 ml Brom und 160 ml Eisessig versetzt. Man läßt mehrere Stunden im Eisbad nachrühren, saugt dann den ausgeschiedenen Niederschlag ab, wäscht ihn mit Wasser und trocknet ihn bei 50 °C. Man erhält 66 g (73 % der Theorie) 3-(3-Amino-2,4,6-tribromphenyl)-2-methoxy-propionsäure als weißes Pulver vom Schmelzpunkt 173-175 °C. 32 g (74 mMol) dieser Verbindung werden bei + 5 °C in 80 ml Nitrosylschwefelsäure portionsweise eingetragen. Nach Zugabe von 40 ml Eisessig läßt man den Ansatz 2 Stunden bei + 5 °C nachrühren, gießt dann auf 360 g Eis auf und trägt ihn in eine Lösung von 33 g Kupfer-I-cyanid, 62 g Kaliumcyanid und 464 ml konzentriertem Ammoniak in 297 ml Wasser ein, wobei Schäumen auftritt. Der Ansatz wird über Nacht bei Raumtemperatur nachgerührt, mit 1 Liter Essigester versetzt und durch Zugabe von konzentrierter Salusäure auf pH 2 gebracht. Nach Filtration über Druckfilter wird die Essig esterphase abgetrennt und die wäßrige Phase erschöpfend mit Essigester nachextrahiert. Die vereinigten Essigesterextrakte werden anschließend über Natriumsulfat getrocknet und nach Filtration über Aktivkohle im Vakuum zur Trockne eingeengt. Man erhält 24 g rohe 3-(3-Cyano-2,4,6-tribrom-phenyl)-2-methoxy-propionsäure, die in 100 ml Wasser nach Zugabe von 3 g Ätznatron bei 60 °C innerhalb 3 Stunden verseift wird. Nach Filtration der erhaltenen Lösung über Aktivkohle wird das Filtrat durch Zugabe von konzentrierter Salzsäure auf pH 2 gebracht und der ausgeschiedene Niederschlag nach mehrstündigem Rühren im Eisbad abgesaugt, mit Wasser gewaschen und bei 50 °C getrocknet. Man erhält 27,5 g (81 % der Theorie) 3-(3-Carbamoyl-2,4,6-tribromphenyl)-2-methoxy-propionsäure als weißes Pulver vom Schmelzpunkt 138-139 °C.

Beispiel 17

Herstellungsbeispiel für Süßungsmittel-Tabletten :

Man mischt 10 kg 3-Carbamoyl-2,4,6-trijodphenoxyessigsäure-Natriumsalz, 40,75 kg Fructose und 0,25 kg Polyoxyethylenpolyoxypropylen-Polymeres mit dem Molgewicht 6800 (Pluronic® F 68) und verarbeitet das Gemisch zu 50 mg Tabletten. Eine Tabelle enthält danach

10 mg 3-Carbamoyl-2,4,6-trijodphenoxyessigsäure
40,75 mg Fructose
0,25 mg Pluronic® F 68.

Beispiel 18

Herstellungsbeispiel für eine Süßungsmittel-Lösung :

Man löst 15 g 3-Carbamoyl-2,4,6-Trijod-phenoxyessigsäure-Natriumsalz und 3 g Gelatine in 250 ml destilliertes Wasser, füllt die klare Lösung mit destilliertem Wasser auf 1 000 ml auf und füllt danach die Lösung in Tropf-Flaschen ab.

**Ansprüche**

1. Süßungsmittel, enthaltend mindestens eine Verbindung der allgemeinen Formel I

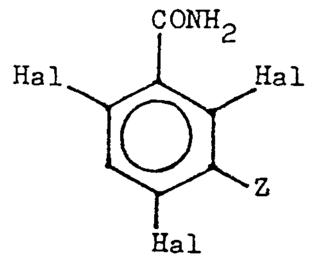

(I)

worin
Hal Chlor, Brom oder Jod ist und
Z eine Carboxylgruppe bedeutet oder die Gruppe

$$-O_P-(CH_2)_K-O_L-(CH_2)_M-(\overset{X}{\overset{|}{C}}H)_N-\overset{Y}{\overset{|}{C}}H-COOH$$

darstellt, wobei
P die Zahl 1 und L die Zahl 0 oder 1 darstellt, jedoch L die Zahl 0 bedeutet, wenn K = 0 ist,
K die Zahlen 0 ; 2 bis 4,
M die Zahlen 0 ; 1 bis 3,
N die Zahlen 0 oder 1,
X ein Wasserstoffatom,
Y ein Wasserstoffatom oder eine niedere Alkylgruppe mit 1 bis 4 Kohlenstoffatomen oder wenn M oder N eine Zahl grösser als 0 ist, auch eine gegebenenfalls verätherte oder veresterte Hydroxygruppe darstellen, oder, wenn N die Zahl 1 bedeutet und M grösser als die Zahl 0 ist,
X und Y zusammen eine zusätzliche Kohlenstoffbindung bilden,
oder wobei P und L die Zahl 0 sind, und
K die Zahlen 0 ; 2 bis 4
M die Zahlen 0 ; 1 bis 3,
N die Zahlen 0 oder 1 bedeuten,
X ein Wasserstoffatom und
Y ein Wasserstoffatom oder eine niedere Alkylgruppe mit 1 bis 4 Kohlenstoffatomen oder eine gegebenenfalls verätherte oder veresterte Hydroxygruppe darstellt, oder, wenn N die Zahl 1 bedeutet, X und Y zusammen eine zusätzliche Kohlenstoffbindung bilden,
sowie deren Salze mit anorganischen Basen.

2. Verwendung von 2,4,6-trihalogenierten Benzamiden der allgemeinen Formel I, wobei Hal, Z, P, K, L, M, N, X und Y die in Anspruch, angegebenen Bedeutungen haben, sowie deren Salze mit anorganischen Basen als Ersatzstoffe für natürliche Süssungsmittel.

3. In 3-Stellung substituierte 2,4,6-trihalogenierte Benzamide der allgemeinen Formel Ia

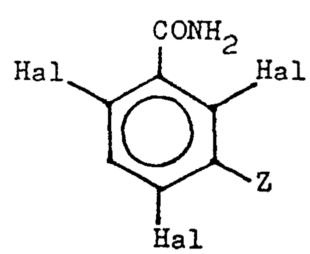

(Ia)

worin

Hal ein Chlor- oder Bromatom ist und

Z eine Carboxylgruppe bedeutet, oder die Gruppe

$$-O_P-(CH_2)_K-O_L-(CH_2)_M-\overset{\overset{X}{|}}{(CH)}_N-\overset{\overset{Y}{|}}{C}H-COOH$$

darstellt, wobei

P und L die Zahlen 0 oder 1 sind, jedoch L die Zahl 0 bedeutet, wenn P oder K = 0 sind,

K die Zahlen 0 ; 2 bis 4,

M die Zahlen 0 ; 1 bis 3,

N die Zahlen 0 oder 1,

X ein Wasserstoffatom,

Y ein Wasserstoffatom oder eine niedere Alkylgruppe mit 1 bis 4 Kohlenstoffatomen oder, wenn M oder N eine Zahl größer 0 sind, Y auch eine gegebenenfalls verätherte oder veresterte Hydroxylgruppe darstellt, oder, wenn N die Zahl 1 bedeutet und M größer als die Zahl 0 ist,

X und Y zusammen eine zusätzliche Kohlenstoffbindung bilden, oder

worin Hal ein Jodatom darstellt, Z eine Carboxylgruppe oder die Gruppe

$$-O_P-(CH_2)_K-O_L-(CH_2)_M-\overset{\overset{X}{|}}{(CH)}_N-\overset{\overset{Y}{|}}{C}H-COOH$$

bedeutet, P und L die Zahlen 0 sind,

und, wenn P und L die Zahl 0 bedeuten

K die Zahlen 0 ; 2 bis 4,

M die Zahlen 0 ; 1 bis 3,

N die Zahlen 0 oder 1,

X ein Wasserstoffatom,

Y ein Wasserstoffatom, eine niedere Alkylgruppe mit 1 bis 4 Kohlenstoffatomen oder eine gegebenenfalls verätherte oder veresterte Hydroxylgruppe darstellt oder wenn N die Zahl I und M grösser als 0 ist,

X und Y zusammen eine zusätzliche Kohlenstoffbindung bilden,

und, wenn P und L die Zahl 1 bedeuten,

K die Zahlen 2 bis 4,

M die Zahlen 0 ; 1 bis 3

N die Zahl 0 oder 1

X ein Wasserstoffatom und

Y ein Wasserstoffatom oder eine niedere Alkylgruppe mit 1 bis 4 Kohlenstoffatomen darstellt,

und, wenn P die Zahl 1 und L die Zahl 0 bedeutet,

K, M und N die Zahl 0 darstellen und

Y ein Wasserstoffatom ist,

und, wenn P die Zahl 1 und L die Zahl 0 bedeutet,

K die Zahl 0,

M die Zahl 1 oder 2,

N die Zahl 0,

Y einen niederen Alkylrest mit 1 bis 4 Kohlenstoffatomen darstellt,

und, wenn P die Zahl 1 und L die Zahl 0 bedeutet,

K die Zahlen 2 bis 4,

M die Zahlen 0 ; 1 bis 3,

N die Zahl 1,

X ein Wasserstoffatom und

Y ein Wasserstoffatom oder einen niederen Alkylrest mit 1 bis 4 Kohlenstoffatomen bedeutet,

sowie deren Salze mit anorganischen Basen.

4. 3-Carbamoyl-2,4,6-trijodphenoxyessigsäure.

5. 3-Carbamoyl-2,4,6-tribromphenoxyessigsäure.

6. 4-(3-Carbamoyl-2,4,6-tribromphenoxy)-buttersäure.

7. 3-Carbamoyl-2,4,6-trichlorphenoxyessigsäure.

8. 4-(3-Carbamoyl-2,4,6-trichlorphenoxy)-buttersäure.

9. 5-(3-Carbamoyl-2,4,6-trichlorphenoxy)-3-oxa-pentansäure.

10. 3-(3-Carbamoyl-2,4,6-tribromphenyl)-propionsäure.

11. 3-(3-Carbamoyl-2,4,6-trichlorphenyl)-propionsäure.
12. 3-(3-Carbamoyl-2,4,6-trijodphenyl)-propionsäure.
13. 3-(3-Carbamoyl-2,4,6-trijodphenyl)-2-äthyl-propionsäure.
14. 3-Carbamoyl-2,4,6-trichlorphenylessigsäure.
15. 3-Carbamoyl-2,4,6-tribromphenyl-essigsäure.
16. 3-(3-Carbamoyl-2,4,6-tribromphenyl)-2-methoxypropionsäure.
17. Verfahren zur Herstellung der Verbindungen der allgemeinen Formel Ia, dadurch gekennzeichnet, daß man

a) eine Verbindung der allgemeinen Formel II

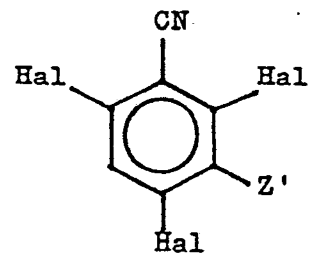

(II)

worin

Hal ein Chlor-, Brom- oder Jodatom ist und

Z' die Gruppe —COOH, —COOR[1] oder

$$-O_P-(CH_2)_K-O_L-(CH_2)_M-(\overset{X}{C}H)_N-\overset{Y}{C}H-COOR^1$$

bedeutet, wobei

$R^1$ ein Wasserstoffatom oder einen niederen Alkylrest mit 1 bis 4 Kohlenstoffatomen darstellt und P, K, L, M, N, X und Y die in Anspruch 1 angegebenen Bedeutungen haben,

in an sich bekannter Weise durch Umsetzung mit Alkali hydrolysiert oder

b) zur Herstellung von Verbindungen der allgemeinen Formel Ia, worin Hal Chlor oder Brom ist und Z die Gruppe

$$-O_P-(CH_2)_K-O_L-(CH_2)_M-(\overset{X}{C}H)_N-\overset{Y}{C}H-COOH$$

darstellt, wobei P die Zahl 1 bedeutet, eine Verbindung der allgemeinen Formel III

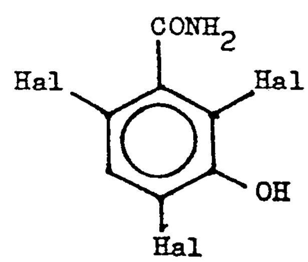

(III)

worin Hal ein Chlor- oder Bromatom darstellt, in an sich bekannter Weise in der Alkalimetallphenolatform umsetzt mit einer Verbindung der allgemeinen Formel IV

$$Hal'-(CH_2)_K-O_L-(CH_2)_M-(\overset{X}{C}H)_N-\overset{Y}{C}H-COOR^1 \quad (IV)$$

worin Hal' ein Chlor- oder Bromatom,

$R^1$ einen niederen Alkylrest mit 1 bis 4 Kohlenstoffatomen darstellt und K, L, M, N, X und Y die in Anspruch 1 angegebenen Bedeutungen besitzen oder zur Herstellung von Verbindungen der allgemeinen Formel Ia, worin Hal Jod ist und Z die Gruppe

$$-O_P-(CH_2)_K-O_L-(CH_2)_M-(\overset{X}{\overset{|}{C}}H)_N-\overset{Y}{\overset{|}{C}}H-COOH$$

darstellt, wobei P die Zahl, bedeutet, eine Verbindung der allgemeinen Formel III, worin Hal ein Jodatom darstellt, in an sich bekannter Weise in der Alkaliphenolatform umsetzt mit einer Verbindung der allgemeinen Formel IV, worin Hal' und $R^1$ die obengenannte Bedeutung haben
und, wenn L die Zahl 1 bedeutet,
K die Zahlen 2 bis 4,
M die Zahlen 0 ; 1 bis 3,
N die Zahl 0 oder 1,
X ein Wasserstoffatom und
Y ein Wasserstoffatom oder eine niedere Alkylgruppe mit 1 bis 4 Kohlenstoffatomen darstellt, oder mit einer Verbindung der allgemeinen Formel IV umsetzt, worin Hal' und $R^1$ die obengenannte Bedeutung haben
und, wenn L die Zahl 0 bedeutet,
K, M und N die Zahl 0 darstellen und
Y ein Wasserstoffatom ist, oder mit einer Verbindung der allgemeinen Formel IV umsetzt, worin Hal' und $R^1$ die obengenannte Bedeutung haben
und, wenn L die Zahl 0 bedeutet,
K die Zahl 0,
M die Zahl 1 oder 2,
N die Zahl 0,
Y einen niederen Alkylrest mit 1 bis 4 Kohlenstoffatomen darstellt, oder mit einer Verbindung der allgemeinen Formel IV umsetzt, worin Hal' und $R^1$ die obengenannte Bedeutung haben
und, wenn L die Zahl 0 bedeutet,
K die Zahlen 2 bis 4,
M die Zahlen 0 ; 1 bis 3
N die Zahl 1,
X ein Wasserstoffatom und
Y ein Wasserstoffatom oder einen niederen Alkylrest mit 1 bis 4 Kohlenstoffatomen bedeutet
und anschließend gegebenenfalls vorhandene Carbonsäurenieder-alkylester verseift und/oder durch Umsetzung mit anorganischen Basen Salze herstellt.

**Claims**

1. Sweetening agent containing at least one compound of the general formula I

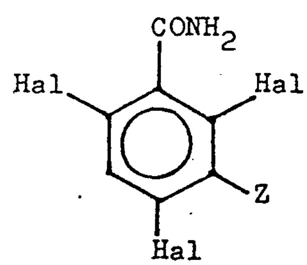

(I)

in which
Hal represents chlorine, bromine or iodine and
Z represents a carboxy group or the group

$$-O_P-(CH_2)_K-O_L-(CH_2)_M-(\overset{X}{\overset{|}{C}}H)_N-\overset{Y}{\overset{|}{C}}H-COOH$$

in which
P represents the number 1 and L represents the number 0 or 1, but
L represents the number 0 when K = 0,
K represents the numbers 0 ; 2 to 4,
M represents the numbers 0 ; 1 to 3,

N represents the number 0 or 1,

X represents a hydrogen atom,

Y represents a hydrogen atom or a lower alkyl group having from 1 to 4 carbon atoms or, when M or N represents a number greater than 0, alternatively an optionally etherified or esterified hydroxy group, or, when N represents the number 1 and M is greater than the number 0,

X and Y together form an additional carbon bond,

or in which

P and L represent the number 0, and

K represents the numbers 0 ; 2 to 4,

M represents the numbers 0 ; 1 to 3,

N represents the number 0 or 1,

X represents a hydrogen atom and

Y represents a hydrogen atom or a lower alkyl group having from 1 to 4 carbon atoms or an optionally etherified or esterified hydroxy group, or, when N represents the number 1, X and Y together form an additional carbon bond,

and also its salts with inorganic bases.

2. Use of 2,4,6-trihalogenated benzamides of the general formula I, in which Hal, Z, P, K, L, M, N, X and Y have the meanings given in claim 1, and also their salts with inorganic bases, as substitutes for natural sweetening agents.

3. 2,4,6-trihalogenated benzamides, substituted in the 3-position, of the general formula Ia

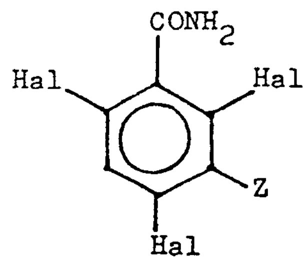

(Ia)

in which

Hal represents a chlorine atom or a bromine atom, and

Z represents a carboxy group, or the group

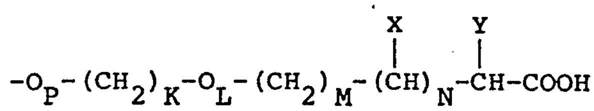

in which

P and L represent the number 0 or 1, but L represents the number 0 when P or K = 0,

K represents the numbers 0 ; 2 to 4,

M represents the numbers 0 ; 1 to 3,

N represents the number 0 or 1,

X represents a hydrogen atom,

Y represents a hydrogen atom or a lower alkyl group having from 1 to 4 carbon atoms or, when M or N represents a number greater than 0, Y alternatively represents an optionally etherified or esterified hydroxy group,

or, when N represents the number 1 and

M is greater than the number 0,

X and Y together form an additional carbon bond,

or in which

Hal represents an iodine atom,

Z represents a carboxy group or the group

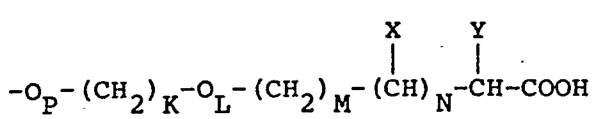

P and L represent the number 0,

K represents the numbers 0 ; 2 to 4,

M represents the numbers 0 ; 1 to 3,

N represents the number 0 or 1,

X represents a hydrogen atom

Y represents a hydrogen atom, a lower alkyl group having from 1 to 4 carbon atoms or an optionally etherified or esterified hydroxy group
or, when N represents the number 1 and M is greater than 0,
X and Y together form an additional carbon bond,
and, when P and L represent the number 1,
K represents the numbers 2 to 4,
M represents the numbers 0 ; 1 to 3,
N represents the number 0 or 1,
X represents a hydrogen atom and
Y represents a hydrogen atom or a lower alkyl group having from 1 to 4 carbon atoms,
and, when P represents the number 1 and L represents the number 0,
K, M and N represent the number 0 and
Y represents a hydrogen atom,
and, when P represents the number 1 and L represents the number 0,
K represents the number 0,
M represents the number 1 or 2,
N represents the number 0,
Y represents a lower alkyl radical having from 1 to 4 carbon atoms,
and, when P represents the number 1 and L represents the number 0,
K represents the numbers 2 to 4,
M represents the numbers 0 ; 1 to 3,
N represents the number 1,
X represents a hydrogen atom and
Y represents a hydrogen atom or a lower alkyl radical having from 1 to 4 carbon atoms,
and also their salts with inorganic bases.

4. 3-carbamoyl-2,4,6-triiodophenoxyacetic acid.
5. 3-carbamoyl-2,4,6-tribromophenoxyacetic acid.
6. 4-(3-carbamoyl-2,4,6-tribromophenoxy)-butyric acid.
7. 3-carbamoyl-2,4,6-trichlorophenoxyacetic acid.
8. 4-(3-carbamoyl-2,4,6-trichlorophenoxy)-butyric acid.
9. 5-(3-carbamoyl-2,4,6-trichlorophenoxy)-3-oxapentanoic acid.
10. 3-(3-carbamoyl-2,4,6-tribromophenyl)-propionic acid.
11. 3-(3-carbamoyl-2,4,6-trichlorophenyl)-propionic acid.
12. 3-(3-carbamoyl-2,4,6-triiodophenyl)-propionic acid.
13. 3-(3-carbamoyl-2,4,6-triiodophenyl)-2-ethyl-propionic acid.
14. 3-carbamoyl-2,4,6-trichlorophenylacetic acid.
15. 3-carbamoyl-2,4,6-tribromophenylacetic acid.
16. 3-(3-carbamoyl-2,4,6-tribromophenyl)-2-methoxypropionic acid.
17. Process for the preparation of the compounds of the general formula Ia, characterised in that

a) a compound of the general formula II

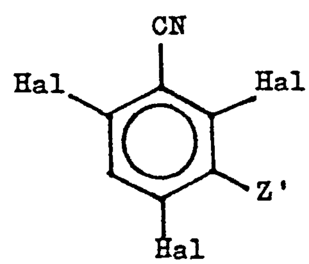

(II)

in which
Hal represents a chlorine, bromine or iodine atom and
Z' represents the group —COOH, —COOR$^1$ or

$$-O_P-(CH_2)_K-O_L-(CH_2)_M-(\overset{X}{C}H)_N-\overset{Y}{C}H-COOR^1$$

in which
R$^1$ represents a hydrogen atom or a lower alkyl radical having from 1 to 4 carbon atoms and P, K, L, M, N, X and Y have the meanings given in claim 1,
is hydrolysed in a manner known per se by reaction with alkali, or

b) for the preparation of compounds of the general formula Ia in which Hal represents chlorine or bromine and Z represents the group

$$-O_P-(CH_2)_K-O_L-(CH_2)_M-(\overset{X}{\overset{|}{C}}H)_N-\overset{Y}{\overset{|}{C}}H-COOH$$

in which P represents the number 1, a compound of the general formula III

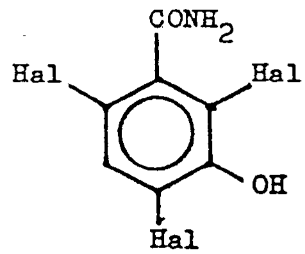

(III)

in which Hal represents a chlorine atom or a bromine atom is reacted in a manner known per se in the alkali metal phenolate form with a compound of the general formula IV

$$Hal'-(CH_2)_K-O_L-(CH_2)_M-(\overset{X}{\overset{|}{C}}H)_N-\overset{Y}{\overset{|}{C}}H-COOR^1 \qquad (IV)$$

in which Hal' represents a chlorine atom or a bromine atom,

$R^1$ represents a lower alkyl radical having from 1 to 4 carbon atoms and K, L, M, N, X and Y have the meanings given in claim 1, or,

for the preparation of compounds of the general formula Ia in which Hal represents iodine and Z represents the group

$$-O_P-(CH_2)_K-O_L-(CH_2)_M-(\overset{X}{\overset{|}{C}}H)_N-\overset{Y}{\overset{|}{C}}H-COOH$$

in which P represents the number 1,

a compound of the general formula III in which Hal represents an iodine atom is reacted in a manner known per se in the alkali phenolate form with a compound of the general formula IV in which Hal' and $R^1$ have the meanings given above,

and, when L represents the number 1,

K represents the numbers 2 to 4,
M represents the numbers 0 ; 1 to 3,
N represents the number 0 or 1,
X represents a hydrogen atom and
Y represents a hydrogen atom or a lower alkyl group having from 1 to 4 carbon atoms,

or with a compound of the general formula IV in which Hal' and $R^1$ have the meanings given above and, when L represents the number 0,

K, M and N represent the number 0 and
Y represents a hydrogen atom,

or with a compound of the general formula IV in which Hal' and $R^1$ have the meanings given above and, when L represents the number 0,

K represents the number 0,
M represents the number 1 or 2,
N represents the number 0,
Y represents a lower alkyl radical having from 1 to 4 carbon atoms,

or with a compound of the general formula IV in which Hal' and $R^1$ have the meanings given above and, when L represents the number 0,

K represents the numbers 2 to 4,
M represents the numbers 0 ; 1 to 3,
N represents the number 1,
X represents a hydrogen atom and

Y represents a hydrogen atom or a lower alkyl radical having from 1 to 4 carbon atoms and then any carboxylic acid lower alkyl esters which may be present are hydrolysed and/or salts are prepared by reaction with inorganic bases.

**Revendications**

1. Edulcorants contenant au moins un composé répondant à la formule générale (I)

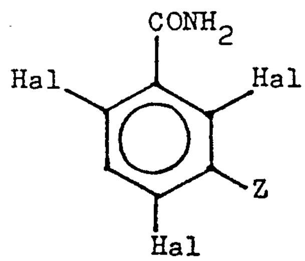

(I)

dans laquelle

Hal représente le chlore, le brome ou l'iode, et

Z représente un radical carboxy ou un radical

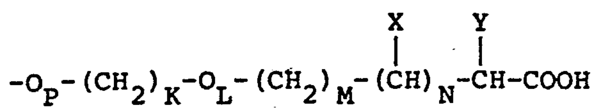

dans lequel

P représente le nombre 1 et L le nombre 0 ou le nombre 1 mais L est égal à 0 dans le cas où K est égal à 0,

K représente l'un des nombres 0 et 2 à 4,

M représente l'un des nombres 0 et 1 à 3,

N représente le nombre 0 ou le nombre 1,

X représente un atome d'hydrogène, et

Y représente un atome d'hydrogène, un radical alkyle inférieur contenant de 1 à 4 atomes de carbone ou, lorsque M ou N désigne un nombre supérieur à 0, Y peut également représenter un radical hydroxy éventuellement éthérifié ou estérifié, ou, lorsque N représente le nombre 1 et que M est un nombre supérieur à 0, X et Y peuvent former ensemble une liaison carbone-carbone supplémentaire,

ou dans lequel

P et L représentent chacun le nombre 0,

K représente l'un des nombres 0 et 2 à 4,

M représente l'un des nombres 0 et 1 à 3,

N représente le nombre 0 ou le nombre 1,

X représente un atome d'hydrogène et

Y représente un atome d'hydrogène, un radical alkyle inférieur contenant de 1 à 4 atomes de carbone ou un radical hydroxy éventuellement éthérifié ou estérifié, ou encore, lorsque N est égal à 1, X et Y peuvent former ensemble une liaison carbone-carbone supplémentaire,

ou un sel formé par un tel composé avec une base minérale.

2. Application de trihalogéno-2,4,6 benzamides de formule générale (I) dans lesquels Hal, Z, P, K, L, M, N, X et Y ont les significations données à la revendication 1, ainsi que des sels qu'ils forment avec des bases minérales, comme succédanés d'édulcorants naturels.

3. Trihalogéno-2,4,6, benzamides substitués en position 3 qui répondent à la formule générale (Ia)

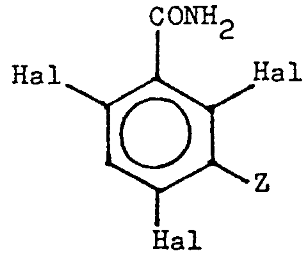

(Ia)

dans laquelle

Hal représente un atome de chlore ou de brome, et
Z représente un radical carboxy ou un radical

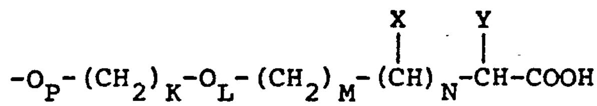

dans lequel

P et L représentent chacun le nombre 0 ou le nombre 1 mais L est égal à 0 lorsque P ou K représente le nombre 0,

K représente l'un des nombres 0 et 2 à 4,

M représente l'un des nombres 0 et 1 à 3,

N représente le nombre 0 ou le nombre 1,

X représente un atome d'hydrogène, et

Y représente un atome d'hydrogène, un radical alkyle inférieur contenant de 1 à 4 atomes de carbone ou, lorsque M ou N est un nombre supérieur à 0, Y peut également représenter un radical hydroxy éventuellement éthérifié ou estérifié, ou, lorsque N est égal à 1 et que M est supérieur au nombre 0, X et Y peuvent former ensemble une liaison carbone-carbone supplémentaire,

ou dans laquelle

Hal représente un atome d'iode et
Z représente un radical carboxy ou un radical

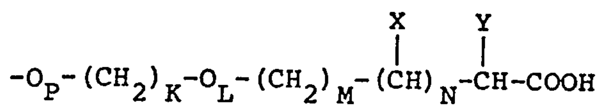

dans lequel

P et L représentent chacun le nombre 0,

K représente l'un des nombres 0 et 2 à 4,

M représente les nombres 0 et 1 à 3,

N représente le nombre 0 ou le nombre 1,

X représente un atome d'hydrogène et

Y représente un atome d'hydrogène, un radical alkyle inférieur contenant de 1 à 4 atomes de carbone ou un radical hydroxy éventuellement éthérifié ou estérifié ou, lorsque N représente le nombre 1 et que M est supérieur à 0, X et Y peuvent former ensemble une liaison carbone-carbone supplémentaire,

et, lorsque P et L représentent chacun le nombre 1,

K représente l'un des nombres 2 à 4,

M représente l'un des nombres 0 et 1 à 3,

N représente le nombre 0 ou le nombre 1,

X représente un atome d'hydrogène, et

Y représente un atome d'hydrogène ou un radical alkyle inférieur contenant de 1 à 4 atomes de carbone,

et, lorsque P représente le nombre 1 et que L représente le nombre 0

K, M et N sont égaux chacun à 0 et

Y représente un atome d'hydrogène,

et, lorsque P représente le nombre 1 et L le nombre 0,

K représente le nombre 0,

M représente le nombre 1 ou le nombre 2,

N représente le nombre 0, et

Y représente un radical alkyle inférieur contenant de 1 à 4 atomes de carbone,

et, lorsque P représente le nombre 1 et L le nombre 0,

K représente l'un des nombres 2 à 4,

M représente l'un des nombres 0 et 1 à 3,

N représente le nombre 1,

X représente un atome d'hydrogène, et

Y représente un atome d'hydrogène ou un radical alkyle inférieur contenant de 1 à 4 atomes de carbone,

ainsi que les sels qu'ils forment avec des bases minérales.

4. Acide (carbamoyl-3 tri-iodo-2,4,6 phénoxy)-acétique.
5. Acide (carbamoyl-3 tri-bromo-2,4,6 phénoxy)-acétique.
6. Acide (carbamoyl-3 tri-bromo-2,4,6 phénoxy)-4 butyrique.
7. Acide (carbamoyl-3 trichloro-2,4,6 phénoxy)-acétique.
8. Acide (carbamoyl-3 trichloro-2,4,6 phénoxy)-4 butyrique.

9. Acide (carbamoyl-3 trichloro-2,4,6 phénoxy)-5 oxa-3 pentanoïque.
10. Acide (carbamoyl-3 tribromo-2,4,6 phényl)-3 propionique.
11. Acide (carbamoyl-3 trichloro-2,4,6 phényl)-3 propionique.
12. Acide (carbamoyl-3 tri-iodo-2,4,6 phényl)-3 propionique.
13. Acide (carbamoyl-3 tri-iodo-2,4,6 phényl)-3 éthyl-2 propionique.
14. Acide (carbamoyl-3 trichloro-2,4,6 phényl)-acétique.
15. Acide (carbamoyl-3 tribromo-2,4,6 phényl)-acétique.
16. Acide (carbamoyl-3 tribromo-2,4,6 phényl)-3 méthoxy-2 propionique.
17. Procédé de préparation des composés de formule générale (Ia), procédé caractérisé en ce que :
a) on hydrolyse un composé répondant à la formule générale (II)

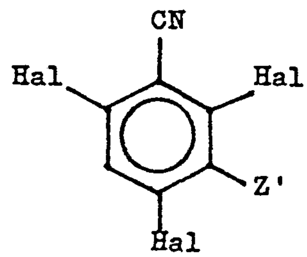

(II)

dans laquelle
Hal représente un atome de chlore, de brome ou d'iode et
Z' représente un radical —COOH, —$COOR^1$ ou

$$-O_P-(CH_2)_K-O_L-(CH_2)_M-(\overset{X}{\overset{|}{C}}H)_N-\overset{Y}{\overset{|}{C}}H-COOR^1$$

où
$R^1$ représente un atome d'hydrogène ou un radical alkyle inférieur contenant de 1 à 4 atomes de carbone, et P, K, L, M, N, X et Y ont les significations données à la revendication 1,
de manière connue, par réaction avec un alcali, ou
b) pour préparer des composés de formule générale (Ia) dans lesquels Hal représente le chlore ou le brome et Z un radical

$$-O_P-(CH_2)_K-O_L-(CH_2)_M-(\overset{X}{\overset{|}{C}}H)_N-\overset{Y}{\overset{|}{C}}H-COOH$$

dans lequel P représente le nombre 1, on fait réagir un composé répondant à la formule générale (III)

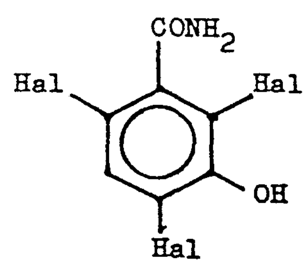

(III)

dans laquelle Hal' représente un atome de chlore ou de brome, de manière connue, sous la forme d'un phénolate de métal alcalin, avec un composé répondant à la formule générale (IV)

$$Hal'-(CH_2)_K-O_L-(CH_2)_M-(\overset{X}{\overset{|}{C}}H)_N-\overset{Y}{\overset{|}{C}}H-COOR^1 \quad (IV)$$

dans laquelle Hal' représente un atome de chlore ou de brome,
$R^1$ représente un radical alkyle inférieur contenant de 1 à 4 atomes de carbone, et K, L, M, N, X et Y

ont les significations données à la revendication 1, ou
pour préparer des composés de formule générale (Ia) dans lesquels Hal représente l'iode et Z représente un radical

$$-O_P-(CH_2)_K-O_L-(CH_2)_M-(\overset{X}{\overset{|}{C}}H)_N-\overset{Y}{\overset{|}{C}}H-COOH$$

dans lequel P représente le nombre 1, on fait réagir un composé de formule générale (III) dans lequel Hal représente un atome d'iode, de manière connue, sous la forme d'un phénolate de métal alcalin, avec un composé de formule générale (IV) dans lequel

Hal' et $R^1$ ont les significations précédemment données,
L représente le nombre 1,
K représente l'un des nombres 2 à 4,
M représente l'un des nombres 0 et 1 à 3,
N représente le nombre 0 ou le nombre 1,
X représente un atome d'hydrogène et
Y représente un atome d'hydrogène ou un radical alkyle inférieur contenant de 1 à 4 atomes de carbone,

ou avec un composé de formule générale (IV) dans lequel :

Hal' et $R^1$ ont les significations précédemment données,
L représente le nombre 0,
K, M et N représentent chacun le nombre 0 et
Y représente un atome d'hydrogène,

ou avec un composé de formule générale (IV) dans lequel :

Hal' et $R^1$ ont les significations précédemment données,
L représente le nombre 0,
K représente le nombre 0,
M représente le nombre 1 ou le nombre 2,
N représente le nombre 0 et
Y représente un radical alkyle inférieur contenant de 1 à 4 atomes de carbone,

ou avec un composé de formule générale (IV) dans lequel :

Hal' et $R^1$ ont les significations précédemment données,
L représente le nombre 0,
K représente l'un des nombres 2 à 4,
M représente l'un des nombres 0 et 1 à 3,
N représente le nombre 1,
X représente un atome d'hydrogène, et
Y représente un atome d'hydrogène ou un radical alkyle inférieur contenant de 1 à 4 atomes de carbone,

après quoi on saponifie d'éventuels radicaux d'esters alkyliques inférieurs d'acides carboxyliques et/ou on prépare des sels par réaction avec des bases minérales.